# EUROPÄISCHE PATENTSCHRIFT

(11) **EP 1 305 097 B1**
(45) Veröffentlichungstag und Bekanntmachung des Hinweises auf die Patenterteilung: **16.06.2004**
(21) Anmeldenummer: 01960573.2
(22) Anmeldetag: 27.07.2001
(51) Int. Cl.: B01D 9/00, G01N 21/51

(54) **REGELUNG EINER WASCHKOLONNE IN EINEM SCHMELZKRISTALLISATIONSPROZESS**
REGULATION OF A WASH COLUMN IN A MELT CRISTALLISATION PROCESS
REGULATION D'UNE COLONNE DE LAVAGE DANS UN PROCEDE DE CRISTALLISATION EN FUSION

(30) Priorität: 28.07.2000 DE 10036880
(43) Veröffentlichungstag der Anmeldung: 02.05.2003
(73) Patentinhaber: BASF AKTIENGESELLSCHAFT, 67056 Ludwigshafen (DE)
(72) Erfinder: HEILEK, Jörg, 69245 Bammental (DE); ECK, Bernd, 68519 Viernheim (DE); BAUMANN, Dieter, 67227 Frankenthal (DE)
(74) Vertreter: Müller, Jörg Uwe
(86) Internationale Anmeldenummer: PCT/EP2001/008712
(87) Internationale Veröffentlichungsnummer: WO 2002/009839

(56) Entgegenhaltungen:
- EP-A- 0 491 558
- WO-A-98/27240
- DE-A- 19 741 307
- GB-A- 2 023 564
- US-A- 4 914 310
- US-A- 5 569 808
- PATENT ABSTRACTS OF JAPAN vol. 010, no. 052 (P-432), 28. Februar 1986 (1986-02-28) & JP 60 195437 A (NIPPON HOSO KYOKAI), 3. Oktober 1985 (1985-10-03)

## Beschreibung

Die vorliegende Erfindung betrifft die Regelung einer Waschkolonne in einem Schmelzkristallisationsprozess, insbesondere die Verwendung eines optischen Zeilendetektors bei einer solchen Regelung, sowie ein entsprechendes Regelverfahren und eine Vorrichtung zur Durchführung des Regelverfahrens.

An die Reinheit von in der chemischen Industrie hergestellten Produkten werden immer höhere Anforderungen gestellt. Dies gilt nicht nur für die sogenannten Feinchemikalien oder für Pharmazeutika, sondern in zunehmendem Maße auch für Massenprodukte, insbesondere für Substanzen, die als Ausgangsmaterialien in der Polymerindustrie verwendet werden, wie beispielsweise Acrylsäure, Caprolactam, *Naphthalin oder Phenol*. *Reinheitsanforderungen von* über 99,99 Gew.% sind für derartige Substanzen nicht ungewöhnlich, da nur hochreine Ausgangsmaterialien eine präzise Kontrolle der Kettenlängenverteilung der Polymere erlauben, die wiederum maßgeblich für die spezifischen Eigenschaften der Polymere ist.

Bei der Synthese einer chemischen Verbindung fällt die gewünschte Substanz jedoch üblicherweise nicht als Reinprodukt an, sondern ist Teil eines Verbindungsgemisches, das neben der gewünschten Substanz Verunreinigungen wie Lösungsmittel, Ausgangsverbindungen, Nebenprodukte oder unerwünschte Isomere enthält. Zur Trennung der gewünschten Substanz von den Verunreinigungen werden im industriellen Maßstab häufig destillative Trennverfahren eingesetzt, die jedoch mit einem hohen Energieaufwand verbunden sind.

Handelt es sich bei der gewünschten Substanz um eine kristallisierbare Verbindung, die nach dem Syntheseprozeß in einem flüssigen Verbindungsgemisch vorliegt, so bietet sich die Schmelzkristallisation als ein mögliches Verfahren zur Reinigung der gewünschten Substanz, d.h. zum Abtrennen der Substanz aus dem flüssigen Verbindungsgemisch an. Dabei wird die gewünschte Verbindung als Feststoff aus der Flüssigkeit auskristallisiert, anschließend der kristalline Feststoff von der restlichen Flüssigkeit, die als Mutterlauge bezeichnet wird, getrennt und wieder aufgeschmolzen. Die Schmelze wird dann als gereinigtes Wertprodukt abgeführt. Übliche Verfahren des Standes der Technik sind die statische und dynamische Schichtkristallisation, bei der die zu isolierende Verbindung an feststehenden, gekühlten Flächen abgeschieden wird, oder die Suspensionskristallisation, die auf dem Wachstum von Kristallen in einer Suspension beruht. Die Suspensionskristallisation weist dabei gegenüber der Schichtkristallisation den Vorteil auf, daß sie in einem kontinuierlichen Prozeß durchgeführt werden kann. Außerdem ist die Reinheit der Kristalle aufgrund ihrer vergleichsweise langsamen Wachstumsgeschwindigkeit sehr hoch. Trotz der langsameren Wachstumsgeschwindigkeit kann mit der Suspensionskristallisation ein hoher Produktdurchsatz erzielt werden, da die Kristallisation in der Lösung mit einer großen für das Wachstum zur Verfügung stehenden Gesamtfläche verbunden ist.

Die Suspensionskristallisation stellt daher ein sehr wirksames und kostengünstiges Verfahren dar, um eine hohe Reinheit der gewünschten Verbindung zu erzielen. Dabei macht man sich zunutze, daß beim Wachstum der Kristalle in einer Flüssigkeit Verunreinigungen weitgehend aus dem Kristallgitter verdrängt werden und in der Mutterlauge zurückbleiben. Bereits in einem einstufigen Kristallisationsprozeß erhält man daher hochreine Kristalle der gewünschten Verbindung.

Der entscheidende Schritt, der die Reinheit des Endproduktes maßgeblich beeinflußt, ist die Abtrennung der hochreinen Kristalle von ihrer Mutterlauge, die die Verunreinigungen und die nicht kristallisierten Anteile des ursprünglichen Gemisches enthält, durch einen Fest/Flüssig-Trennprozeß. Dieser Trennprozeß kann mehrstufig ablaufen, wobei zumindest in der letzten Stufe üblicherweise eine sogenannte Waschkolonne verwendet wird. Die Waschkolonne hat die Aufgabe, die anfallende reine Kristallphase möglichst vollständig von der Mutterlauge zu trennen. Dazu wird die in einem Kristallisator erzeugte Kristallsuspension in die Waschkolonne eingeleitet und durch Mutterlaugenentzug ein dichteres Kristallbett erzeugt. Eine Waschflüssigkeit, beispielsweise eine Schmelze aus den aufgeschmolzenen Kristallen selbst, wird im Gegenstrom durch das Kristallbett geleitet.

Zur Ausbildung eines kompakten Kristallbetts werden unterschiedliche Methoden eingesetzt. Bei gravitativ arbeitenden Waschkolonnen wird die Kristallsuspension von oben in die Kolonne eingeführt und das Kristallbett bildet sich in einem Sedimentationsprozeß aus. Bei derartigen Kolonnen besteht jedoch die Gefahr, dass sich im Laufe des Sedimentationsprozesses vertikale Kanäle ausbilden, in denen eine Rückvermischung der Mutterlauge oder der Kristallsuspension mit der Waschflüssigkeit auftritt. Daher sind gravitativ arbeitende Waschkolonnen auf einem Teil ihrer Höhe meist mit einem Rührwerk versehen, das die Ausbildung von vertikalen Flüssigkeitskanälen im Kristallbett verhindert.

Derartige Rührwerke sind bei hydraulischen oder mechanischen Waschkolonnen nicht erforderlich. Bei hydraulischen Waschkolonnen wird die Suspension vielmehr unter Druck in eine druckdicht ausgebildete Waschkolonne gefördert. Der Förderdruck selbst sorgt dann für eine Kompaktierung der Kristalle zu einem dichten Festbett. Bei einer mechanischen Waschkolonne wird der Druck zur Ausbildung eines dichten Kristallbetts beispielsweise durch einen mechanischen, semipermeablen Stempel erzeugt, der für Mutterlauge durchlässig, aber für die Kristalle in der zugeführten Suspension undurchlässig ist. Die Verdichtung zu einem Kristallbett kann aber auch durch Abtrennung der Mutterlauge über Filter und mechanischen Transport der Kristalle vom Filter zum Kristallbett durch ein rotierendes Förderelement erfolgen.

Das Kristallbett weist eine sog. Aufbaufront auf, an der sich kontinuierlich Kristalle der eingeleiteten Kristallsuspension anlagern. Die Aufbaufront bezeichnet also den Übergang von der Suspension zum Kristallbett und ist durch einen relativ abrupten Anstieg des Kristallgehalts in der Suspension gekennzeichnet. Bei hydraulischen Waschkolonnen wird diese Aufbaufront auch als Filtrationsfront bezeichnet.

An dem der Aufbaufront gegenüber liegenden Ende des Kristallbettes ist meist eine Art Rotormesser oder Schaber angeordnet, der kontinuierlich Kristalle vom dichten Kristallbett abträgt. Durch die kontinuierliche Anlagerung von Kristallen an der Aufbaufront einerseits und das kontinuierliche Abtragen von Kristallen an dem der Aufbaufront gegenüber liegenden Ende des Kristallbetts andererseits, wird eine Transportrichtung des Kristallbettes definiert. Die vom Kristallbett abgetragenen Kristalle werden in einem Wärmeübertrager aufgeschmolzen. Ein Teil der Schmelze wird als Reinproduktstrom abgeführt und ein anderer Teil der Schmelze als Waschflüssigkeitsstrom gegen die Transportrichtung der Kristalle durch das Kristallbett geleitet.

Durch die Förderung der Schmelze entgegen dem Kristallbett erfolgt eine Gegenstromwäsche der Kristalle. Die Reinigung der Kristalle beruht dabei im Wesentlichen auf einer Verdrängung und Verdünnung der Mutterlauge in den Zwickeln des Kristallbettes durch die Waschflüssigkeit. Der Verdünnungseffekt beruht hierbei auf Vermischung in den durchströmten Zwickeln zwischen den Kristallen und auf Diffusion in den nicht durchströmten Kontaktstellen bzw. der oberflächennahen Strömungsgrenzschicht der Kristalle. Im stationären Betrieb stellt sich auf einer definierten Höhe des Kristallbetts eine sog. Waschfront ein, die als derjenige Ort in der Waschkolonne definiert ist, wo die höchsten Temperatur- und Konzentrationsgradienten auftreten. Auf Höhe der Waschfront findet nämlich in der die Kristalle umgebenden Flüssigkeit ein Konzentrationsübergang von Mutterlaugenkonzentration (oberhalb der Waschfront) zu Reinschmelzekonzentration (unterhalb der Waschfront) statt. Die Waschfront muß zur Erzielung einer adäquaten Reinigungswirkung in einer bestimmten Mindesthöhe oberhalb des Schabers positioniert sein. Da die Kristallisationstemperatur in der verunreinigten Suspension unterhalb des Reinproduktschmelzpunktes liegt, kommt es im Bereich der Waschfront außerdem zu einem Temperaturausgleich der kalten Kristalle mit der reinen Waschflüssigkeit, bei dem die Waschflüssigkeit teilweise oder vollständig rekristallisiert. Dadurch kann zumindest ein Teil der Waschflüssigkeit zurückgewonnen werden. Diese Rekristallisation der Waschflüssigkeit ist besonders wirksam, wenn die Kristallisationstemperatur in der Mutterlauge ca. 10 bis 15 K unterhalb der Schmelztemperatur des Reinprodukts liegt.

Zur Gewährleistung eines stabilen Betriebs einer Waschkolonne, d.h. zur Gewährleistung einer definierten Raum-Zeit-Ausbeute bei konstant guter Reinigungswirkung, ist eine kontinuierliche Kompensation äußerer Störgrößen erforderlich. Derartige Störgrößen können beispielsweise Schwankungen der Suspensionsmenge, Änderungen des Kristallgehalts in der Suspension, Variation der Kristallgrößenverteilung oder auch Konzentrationsschwankungen in dem, dem Kristallisator zugeführten Produktgemisch aus dem Syntheseprozeß sein.

Die Kompensation derartiger äußerer Störungen erfolgt üblicherweise:
durch Regelung der Schmelzwärme;
durch Adaption der spezifischen Waschflüssigkeitsmenge mittels Regelung der Lage der Waschfront;
sowie bei hydraulischen und gravitativen Waschkolonnen zusätzlich durch Regelung der Lage der Aufbaufront.

Zusätzlich kann zur Kontrolle die Reinheit der Reinproduktschmelzen in einer Produktabzugsleitung oder in einer Leitung des Schmelzkreislaufs beispielsweise ein Extinktionssensor angeordnet sein, der die Extinktion in einem für das gewünschte Produkt charakteristischen Spektralbereich bestimmt. Ist der Extinktionssensor in einer Leitung des Schmelzkreislaufs angeordnet, kann er auch für das Anfahren der Waschkolonne genutzt werden, so daß beim Anfahren der Zeitpunkt bestimmt werden kann, bei dem das Produktventil erstmals geöffnet wird.

Die Regelung der Schmelzwärme, d.h. der Eintrag der zum Schmelzen der Kristalle im Schmelzkreislauf erforderlichen Wärmemenge wird üblicherweise durch Regelung der Produkttemperatur nach dem Wärmeübertrager sichergestellt. Die Temperatur im Schmelzkreislauf unmittelbar nach dem Wärmeübertrager liegt dabei bevorzugt ca. 1 - 5 K über dem Schmelzpunkt des Reinprodukts. Das Rotormesser oder der Schaber wird üblicherweise mit fest eingestellter Drehzahl betrieben. Geeignete Verfahren zur Regelung der Schmelzwärme sind dem Fachmann bekannt und sind nicht Gegenstand der vorliegenden Erfindung.

Die vorliegende Erfindung betrifft vielmehr die Regelung der Lage der Waschfront und/oder der Lage der der Aufbaufront des *Kri*stallbetts.

Eine konstante Lage der Waschfront im Kristallbett gewährleistet, daß eine ausreichend große Waschflüssigkeitsmenge im Gegenstrom zur Transportrichtung des Kristallbetts strömt, so daß eine bestimmte Reinheit des Endproduktes erzielt werden kann. Als spezifische Waschflüssigkeitsmenge bezeichnet man dabei die innerhalb eines bestimmten zeitintervalls zur Erzielung einer definierten Trennwirkung aufzuwendende Waschflüssigkeitsmenge, bezogen auf die der Waschkolonne in diesem Zeitintervall zugeführte Kristallmenge. Herkömmlicherweise wird die spezifische Waschflüssigkeitsmenge durch Regelung der Lage der Waschfront unterhalb des Filters in der Kolonne eingestellt. Die Waschfront wird dabei durch Einstellung der Waschflüssigkeitsmenge über das Produktventil auf einer definierten Position zwischen dem Filter und dem Schaber eingeregelt. Damit ist gewährleistet, daß eine gewünschte Trennwirkung, d.h. eine bestimmte Produktreinheit, mit minimalem Aufwand an Waschflüssigkeit erfüllt wird. Zur Detektion der Waschfront können beispielsweise ein oder mehrere im Kristallbett angeordnete Temperatursensoren verwendet werden, da auf Höhe der Waschfront der Temperaturübergang von der Kristallisationstemperatur zur Schmelztemperatur des Reinprodukts erfolgt. So beschreibt die GB-A-2 023 564 eine Vorrichtung zur Trennung von Kristallen und Mutterlauge in einem Suspensionskristallisationsprozeß mit einer Temperaturmesseinrichtung zur Bestimmung der Lage einer Aufbaufront oder einer Waschfront des Kristallbetts in der Waschkolonne und Mitteln zur Regelung der Lage der Waschfront. Alternativ können auch optische Sensoren, beispielsweise Remissionssonden, zur Detektion der Lage der Waschfront herangezogen werden. Oberhalb der Waschfront besteht die das Kristallbett umgebende Flüssigkeit im wesentlichen aus verunreinigter Mutterlauge und unterhalb der Waschfront dagegen aus Reinproduktschmelze. Je nach Art der beteiligten Substanzen kann es bei diesem Übergang zu meßbaren Änderungen der optischen Eigenschaften, beispielsweise der Remissionseigenschaften kommen. Herkömmlicherweise wird, wie bei der Regelung der Aufbaufront, die Waschfront zwischen zwei axial beabstandeten Remissionssensoren eingeregelt.

Eine konstante Lage der Aufbaufront gewährleistet zu jedem Zeitpunkt die Einhaltung der äußeren Massenbilanz der Waschkolonne, d.h. es ist in diesem Fall gewährleistet, dass die gleiche Menge aufgeschmolzenes Reinprodukt abgezogen wird bzw. als Verlust mit der Mutterlauge die Waschkolonne verläßt, wie an Kristallen neu in die Waschkolonne gelangt. Die Lage der Aufbaufront in der Waschkolonne wird üblicherweise mit Hilfe von zwei optischen Remissionssensoren bestimmt, die in einem gewissen axialen Abstand voneinander (d.h. gemessen entlang der Längsachse der Waschkolonne) in einer definierter Höhe in der zylindrischen Seitenwand der Waschkolonne angeordnet sind. Die Lage der Aufbau- bzw. Filtrationsfront kann beispielsweise bei hydraulischen Waschkolonnen durch eine Einstellung der hydraulischen Verhältnisse in der Waschkolonne beeinflußt werden. Wenn beispielsweise kontinuierlich Mutterlauge über entsprechende Filter aus der Waschkolonne abgezogen wird, bietet es sich an, einen Teil dieser abgezogenen Mutterlauge zur Beeinflussung des hydrodynamischen Drucks in der Kolonne in diese zurückzupumpen. Eingestellt wird dabei die zurückgeführte Mutterlaugenmenge, die mit einer entsprechenden Steuerstrompumpe z.B. durch Drehzahländerung variiert werden kann. Zeigt beispielsweise der obere Remissionssensor einen Anstieg des Kristallbettes in der Waschkolonne an, so sorgt ein Regelkreis für eine Erhöhung der Steuerstrommenge. Entsprechend wird die Steuerstrommenge bei absinkendem Bett reduziert. Die Änderung der Steuerstrommenge wird dabei nach einer definierten Charakteristik durchgeführt, z.B. linear in Abhängigkeit vom Mengenstrom und der zeit.

Die beiden Remissionssensoren, die üblicherweise zur Regelung der Wasch- oder Aufbaufront herangezogen werden, weisen bei Waschkolonnen mit einem ca. 1 m hohen Kristallbett typischerweise einen axialen Abstand (d.h. gemessen entlang der Längsachse der Waschkolonne) von 5 bis 10 cm auf. Dieser Abstand muß einerseits ausreichend groß gewählt werden, damit die Signaldifferenz zwischen den von den beiden Sensoren gelieferten Signalen groß genug für einen Regelung ist. Andererseits entspricht der Abstand der beiden Remissionssensoren auch die Genauigkeit, mit der die Lage der Aufbaufront geregelt werden kann. Das bekannte Regelungskonzept mit Hilfe von zwei axial beabstandeten optischen Sensoren, weist den Nachteil auf, daß die Lage der Fronten nur sehr ungenau innerhalb eines von zwei Randwerten definierten, relativ breiten Bereichs geregelt werden kann. Dieser Nachteil macht sich insbesondere dann bemerkbar, wenn die an der Aufbaufront oder an der Waschfront auftretenden optischen Änderungen gering sind. Dies ist beispielsweise beim Anfahren einer Waschkolonne der Fall, wenn sich die Fronten im Laufe der Zeit erst ausbilden, aber beispielsweise auch im laufenden Betrieb, wenn ein bereits relativ reines Ausgangsprodukt durch Schmelzkristallisation weiter gereinigt werden soll. Außerdem ist die bekannte 2-Punkt-Regelung relativ instabil, so daß größere Störungen leicht zu einem Versagen des Regelung führen können.

Aufgabe der vorliegenden Erfindung ist es daher, ein Verfahren und eine Vorrichtung zur Regelung einer Waschkolonne in einem Schmelzkristallisationsprozeß anzugeben, wobei die Lage der Waschfront und/oder der Aufbau- bzw. Filtrationsfront mit größerer Genauigkeit geregelt werden kann. Außerdem soll eine zuverlässige Regelung auch dann ermöglicht werden, wenn an der Waschfront oder der Aufbaufront nur geringe Änderungen der optischen Eigenschaften des Kristallbettes bzw. der das Kristallbett umgebenden Flüssigkeit auftreten.

Gelöst wird diese Aufgabe durch Verwendung eines optischen Zeilendetektors zur Regelung der Lage der Waschfront und/oder der Aufbaufront in einer Waschkolonne bei einem Schmelzkristallisationsprozeß. Der Zeilendetektor ist erfindungsgemäß so angeordnet, daß optische Eigenschaften des Kristallbettes in einem parallel zur Längsachse der Waschkolonne verlaufenden Bereich kontinuierlich erfaßt werden können, wobei dieser Bereich die gewünschte Sollposition der Lage der Waschfront bzw. der Lage der Aufbaufront überdeckt. Der zeilendetektor weist mehr als zwei, vorzugsweise mehr als vier und besonders bevorzugt mehr als 10 Meßelemente (Sensorzellen) auf, so daß der axiale Verlauf der untersuchten optischen Eigenschaften und damit auch die Lage der Aufbau- oder Waschfront wesentlich präziser als mit der bekannten 2-Punkt-Messung bestimmt werden kann.

Gegenstand der vorliegenden Erfindung ist demnach auch ein Verfahren zur Regelung einer Waschkolonne in einem Schmelzkristallisationsprozeß, bei dem man eine Suspension, die in einer Mutterlauge suspendierte Kristalle einer zu reinigenden Substanz enthält, kontinuierlich in eine Waschkolonne leitet, in der Waschkolonne ein Kristallbett der zu reinigenden Substanz ausbildet, wobei das Kristallbett eine Aufbaufront aufweist, an der sich kontinuierlich Kristalle der eingeleiteten Suspension anlagern, an dem der Aufbaufront gegenüberliegenden Ende des Kristallbetts kontinuierlich Kristalle abträgt, die abgetragenen Kristalle aufschmilzt, einen Teil der Schmelze als Reinproduktstrom abführt und einen anderen Schmelze als Waschflüssigkeitsstrom gegen die Transportrichtung der Kristalle durch das Kristallbett leitet, wobei sich im Kristallbett eine Waschfront ausbildet. Das Verfahren ist erfindungsgemäß dadurch gekennzeichnet, daß man die Lage der Aufbaufront und/oder die Lage der Waschfront in der Waschkolonne mittels wenigstens eines optischen zeilendetektors regelt.

Als optische Zeilendetektoren kommen unterschiedlichste lineare Sensorarrays mit mehr als zwei Sensorelementen in Frage, welche in der Lage sind, die gewünschten optischen Eigenschaften des Kristallbetts zu bestimmen. Bei einer typischen Kristallbetthöhe zwischen Aufbaufront und Schaber von 0,5 m bei einer mechanische Waschkolonne, von 0,5 bis 1,5 m bei einer hydraulischen Waschkolonne und bis zu 5 m bei einer gravitativen Waschkolonne beträgt der von dem Zeilendetektor zu überwachende Regelbereich typischerweise 5 bis 30, bevorzugt 5 bis 10 cm. Die Signale der einzelnen Sensorelemente des Zeilendetektors werden digitalisiert und einer Auswerteeinrichtung, beispielsweise einem Computer, übermittelt. Die Auswerteeinrichtung analysiert die vom Zeilendetektor registrierten Daten. Als Lage der Aufbau- oder Waschfront kann man beispielsweise die Position bestimmen, bei der das gemessene optische Signal die größte Änderung zeigt (also den Wendepunkt des Signalverlaufs).Die Präzision der Regelung ist also nicht mehr von einer minimalen Signaldifferenz zwischen benachbarten Sensoren abhängig und ist daher wesentliche höher als bei der konventionellen 2-Punkt-Regelung.

Neben der höheren Genauigkeit der Regelung der Lage der Waschoder Aufbaufront, ist ein besonderer Vorteil der Erfindung darin zu sehen, daß die Nutzung mehrerer Sensorelemente eine stabilere Regelung erlaubt. Je mehr Sensorelemente nämlich den zu überwachenden Bereich abdecken, desto eher kann man mit konventionellen Regelungskonzepten, wie einer PI-Regelung (Proportional-Integral-Regelung) arbeiten. Im Gegensatz zur konventionellen 2-Punkt-Regelung lassen sich so auch Driftanteile im Meßsignal kompensieren, die beispielsweise durch Änderung der Lampenintensität oder durch Ablagerungen auf der Sonsorfläche oder auf einem Meßfenster in der Kolonnenwand hervorgerufen werden.

Zur Regelung der Lage der Waschfront wird die momentane Position der Waschfront vorteilhaft mittels eines optischen Zeilendetektors gemessen und durch entsprechende Steuerung des abgezogenen Reinproduktstroms auf eine Sollposition geregelt.

Wenn es sich bei der im erfindungsgemäßen Verfahren verwendeten Waschkolonne um eine hydraulische Waschkolonne handelt, kann man auch die Lage der Aufbaufront mittels eines optischen Zeilendetektors bestimmen und durch Steuerung des hydrodynamischen Drucks in der Waschkolonne, beispielsweise durch Rückführung von Mutterlauge mittels einer Steuerstrompumpe, auf eine Sollposition regeln.

Gemäß einer bevorzugten Variante des erfindungsgemäßen Verfahrens verwendet man als optischen Zeilendetektor wenigstens ein lineares Array von faseroptischen Remissionssonden. Jede dieser faseroptischen Remissionssonden kann beispielsweise eine oder mehrere Sendefasern und eine oder mehrere Empfangsfasern aufweisen. Die Sendefasern sind mit einem Faserende mit einer Lichtquelle verbunden, so daß Anregungslicht in die Fasern eingekoppelt werden kann. Das eingekoppelte Licht kann am anderen Ende der Sendefasern austreten. Die Remissionssonden sind dabei so am zylindrischen Mantel der Waschkolonne angeordnet, daß das aus den Sendefasern austretende Licht in das Kristallbett fällt und teilweise zur Sonde zurückgestreut wird. In jeder Sonde befinden sich auch Empfangsfasern, die zurückgestreutes Licht auffangen und zu einem Detektorsystem leiten. Anstelle von getrennten Sende- und Empfangsfasern kann man auch eine Faser verwenden, die sowohl als Sende- als auch als Empfangsfaser wirkt. An einem Faserende ist dann jedoch eine Y-förmige Verzweigung zur Lichtquelle bzw. zum Detektor vorgesehen. Die einzelnen linear angeordneten Remissionssonden sind nicht notwenigerweise baulich getrennte Einheiten. Mit dem Begriff Remissionssonde ist vielmehr eine Gruppe von einander zugeordneten Sende- und Empfangsfasern gemeint, die gemeinsam ein Meßelement des linearen Arrays bilden. So kann jede einzelne Remissionssonde ein Meßelement von beispielsweise 1 mm² Fläche bilden. Mit einem linearen Array von 10 derartigen, im Abstand von 1 cm angeordneten Remissionssonden kann die zu regelnde Front mit also einer Genauigkeit von 1 cm stabil geregelt werden kann.

Gemäß einer anderen Variante des erfindungsgemäßen Verfahrens verwendet man als optischen Zeilendetektor wenigstens eine zeilenkamera, insbesondere eine CCD-Zeilenkamera. In diesem Fall sind in der zylindrischen Wand der Waschkolonne ein oder mehrere längliche Sichtfenster angeordnet, die über eine Abbildungsoptik der Kamera auf den CCD-Zeilenchip abgebildet werden. In diesem Fall wird üblicherweise auch eine Beleuchtungseinrichtung verwendet, die das Sichtfenster und das dahinter liegende Kristallbett ausleuchtet.

Je nach optischen Eigenschaften der in der Waschkolonne aufzutrennenden Materialien kann man mit Hilfe geeigneter Filter, die sowohl in der Beleuchtungseinrichtung, als auch im Detektor der Remissionssonde bzw. in der Abbildungsoptik der Zeilenkamera angeordnet sein können, Spektralbereiche selektieren, bei denen besonders große Signaländerungen erwartet werden können. Es ist auch möglich, beispielsweise eine CCD-Farbkamera zu verwenden, bei der jedes Meßelement durch drei CCD-Chips mit unterschiedlicher spektraler Empfindlichkeit repräsentiert wird. Bei der Schmelzkristallisationsreinigung von Acrylsäure wird beispielsweise die Lage der Waschfront bevorzugt durch Messung der Remission in einem Spektralbereich zwischen 300 und 400 nm bestimmt.

Gegenstand der vorliegenden Erfindung ist auch eine Vorrichtung zur Trennung von Kristallen und Mutterlauge in einem Suspensionskristallisationsprozeß, die insbesondere zur Durchführung des erfindungsgemäßen Verfahrens geeignet ist. Die Vorrichtung umfaßt eine Waschkolonne, die wenigstens eine Zuleitung für eine Kristallsuspension, Filtrationsmittel zum Abführen von Mutterlauge und Mittel zum Abtragen von Kristallen aus einem Kristallbett aufweist, Mittel zum Aufschmelzen der abgetragenen Kristalle, eine Abzugseinrichtung zum Abführen eines Teils der Schmelze als Reinproduktstrom, wenigstens eine Meßeinrichtung zur Bestimmung der Lage einer Aufbaufront und/oder einer Waschfront des Kristallbettes in der Waschkolonne und Mittel zur Regelung der Lage der Aufbaufront und/oder der Lage der Waschfront, wobei die erfindungsgemäße Vorrichtung dadurch gekennzeichnet ist, daß die Meßeinrichtung wenigstens einen optischen zeilendetektor umfaßt.

Gemäß einer Ausführungsform der erfindungsgemäßen Vorrichtung umfaßt der optische Zeilendetektor ein lineares Array von mehr als zwei, bevorzugt mehr als vier und besonders bevorzugt mehr als 10 faseroptischen Remissionssonden, die in einem Mantel der Waschkolonne angeordnet sind.

Gemäß einer anderen Ausführungsform ist im Mantel der Waschkolonne wenigstens ein transparentes Fenster angeordnet und der optische Zeilendetektor umfaßt wenigstens eine Zeilenkamera, welche auf das transparente Fenster ausgerichtet ist. Besonders bevorzugt ist die Zeilenkamera eine CCD-Zeilenkamera, beispielsweise eine Farb-CCD-Zeilenkamera. Derartige Zeilenkameras sind kommerziell erhältlich und weisen typischerweise 1024 oder 2048 linear angeordnete Pixel auf. Die Fläche eines Pixels liegt typischerweise in der Größenordnung von 50 bis 100 µm².

Das erfindungsgemäße Verfahren und die erfindungsgemäße Vorrichtung eignen sich für alle Arten von Waschkolonnen, bei denen im Betrieb eine Waschfront und/oder eine Aufbau- bzw. Filtrationsfront entsteht, also insbesondere für hydraulische, mechanische oder gravitative Waschkolonnen. Bevorzugt wird die Erfindung in Schmelzkristallisationsprozessen angewandt, bei denen eine Schmelze des Reinproduktes als Waschflüssigkeit verwendet wird, jedoch ist das erfindungsgemäße Regelungsprinzip auch dann anwendbar, wenn andere Flüssigkeiten als eine Produktschmelze als Waschflüssigkeit verwendet werden.

Die vorliegende Erfindung wird im folgenden unter Bezugnahme auf in den beigefügten Zeichnungen dargestellte Ausführungsbeispiele ausführlicher erläutert.

In den Zeichnungen zeigt:
- Figur 1: eine schematische Übersichtsdarstellung eines Suspensionskristallisationsprozesses;
- Figur 2: eine erste Ausführungsform einer erfindungsgemäßen Vorrichtung;
- Figur 3: eine zweite Ausführungsform einer erfindungsgemäßen Vorrichtung;
- Figur 4: eine Variante der Vorrichtung der Figur 3.

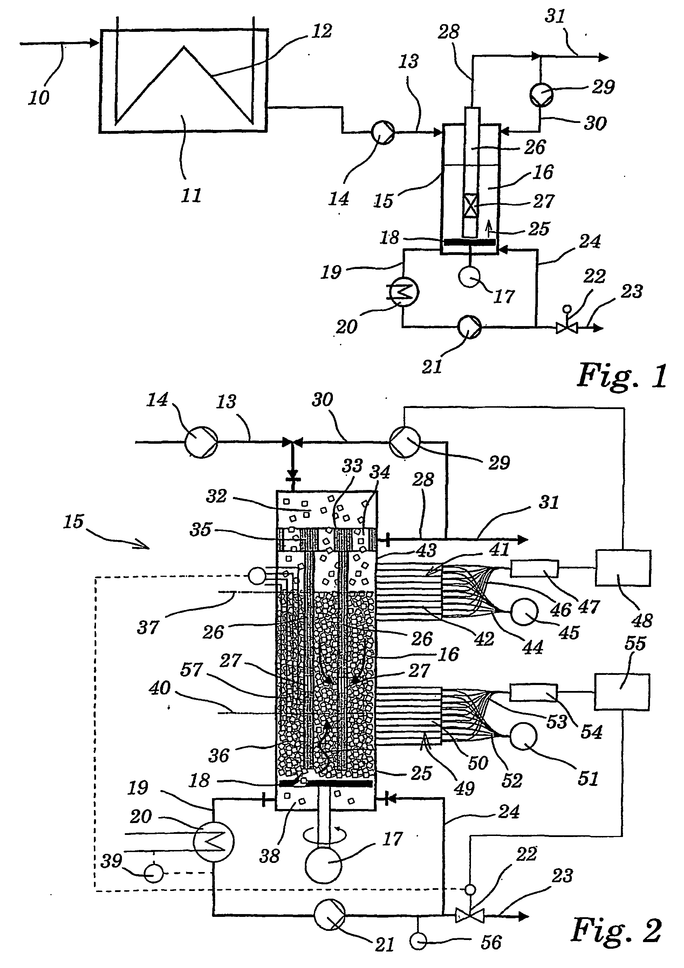

In Figur 1 ist der schematische Aufbau einer an sich bekannten Anlage zur Reinigung von Syntheseprodukten durch Suspensionskristallisation dargestellt. Ein aus einem (nicht dargestellten) Syntheseprozeß stammendes flüssiges Produktgemisch wird über eine Leitung 10 einem Kristallisator 11 zugeführt. In dem Kristallisator 11 befindet sich ein Wärmeübertrager 12, der dem Produktgemisch Wärme entzieht. In der Flüssigkeit beginnen Kristalle der gewünschten Verbindung zu wachsen. Die im Kristallisator 11 entstandene Kristallsuspension (Kristalle und Mutterlauge) wird mittels einer in einer Verbindungsleitung 13 angeordneten Pumpe 14 in eine Waschkolonne 15 gefördert, die im dargestellten Beispiel als hydraulische Waschkolonne ausgebildet ist. Die Funktionsweise der Waschkolonne 15 wird unten im Zusammenhang mit der detaillierteren Darstellung der Figur 2 näher erläutert. Im wesentlichen werden die Kristalle der zugeführten Suspension in der Waschkolonne 15 zu einem dichten Kristallbett kompaktiert, das im Fall der dargestellten hydraulischen Waschkolonne als Festbett 16 ausgebildet ist. Am unteren Ende des Festbettes 16 ist ein durch einen Motor 17 angetriebener Schaber 18 angeordnet, der kontinuierlich Kristalle von dem Festbett abträgt. Die Kristalle gelangen in einen Schmelzkreislauf 19, in welchem ein Wärmeübertrager 20 und eine Pumpe 21 angeordnet sind und werden dort aufgeschmolzen. Über ein einstellbares Produktventil 22 wird ein Teil der Schmelze als gewünschtes Reinprodukt durch eine Leitung 23 aus dem Schmelzkreislauf 19 abgezogen. Der andere Teil der Schmelze wird über einen Leitungsabschnitt 24 des Schmelzkreislaufs 19 in die Waschkolonne 15 zurückgeleitet und kann das Festbett 16 teilweise als Waschflüssigkeit im Gegenstrom zur Transportrichtung der Kristalle durchströmen. Die Strömungsrichtung der Waschflüssigkeit ist in Fig. 1 durch einen Pfeil 25 symbolisiert.

In der Waschkolonne 15 sind ein oder mehrere vertikale Drainagerohre 26 angeordnet, die jeweils auf definierter Höhe mit einem Filter 27 versehen sind. Über den Filter 27 wird im wesentlichen die Mutterlauge, aber gegebenenfalls auch ein Teil der als Waschflüssigkeit vom unteren Bereich der Kolonne zu den Filtern strömenden Schmelze (Pfeil 25) oder sehr kleine Kristallite, welche die Filter passieren können, über eine Leitung 28 aus der Waschkolonne 15 abgezogen. Größere Kristalle können die Filter 27 aber nicht passieren. Ein Teil der über die Leitung 28 die Waschkolonne 15 verlassenden Mutterlauge wird mittels einer Steuerstrompumpe 29 über eine Rückflußleitung 30 in den oberen Bereich der Waschkolonne 15 zurückgeführt. Dadurch ist es möglich, die hydraulischen Bedingungen in der Waschkolonne 15 zu regulieren. Die übrige abgezogene Flüssigkeit fließt über eine Leitung 31 ab.

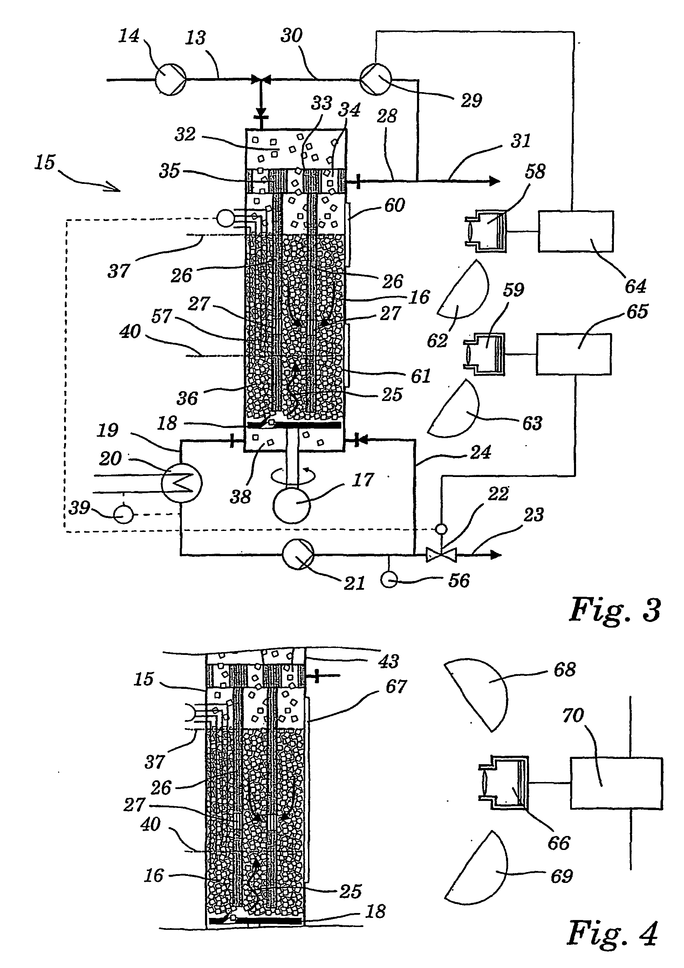

In Fig. 2 ist der Aufbau einer ersten Ausführungsform einer erfindungsgemäßen Waschkolonne, wie sie in der Anlage der Fig. 1 eingesetzt werden kann, detaillierter dargestellt. Elemente und Bauteile, die bereits im Zusammenhang mit Fig. 1 erläutert wurden, sind mit denselben Bezugziffern bezeichnet und werden nicht mehr ausführlicher beschrieben. Im dargestellten Beispiel, ebenso wie in den Beispielen der folgenden Figuren 3 und 4, ist die Waschkolonne 15 als hydraulische Waschkolonne ausgebildet. Die aus dem Kristallisator über die Leitung 13 abgezogene Kristallsuspension 32 wird mittels der Pumpe 14 (oder über hydrostatischen Druck) in die Waschkolonne 15 eingespeist. Im oberen Teil der hydraulischen Waschkolonne ist ein Fluidregister 33 angeordnet, das zwei Funktionen erfüllt: Über Durchgangsöffnungen 34 vom oberen zum unteren Kolonnenteil wird die Suspension 32 über den Querschnitt der Waschkolonne 15 verteilt. Der zusammenhängende Innenraum 35 des Fluidregisters dient als Sammler für die abgeführten Flüssigkeiten, insbesondere Mutterlauge und Waschflüssigkeit. Dazu sind am unteren Ende des Fluidregisters 33 die bereits oben erwähnten Drainagerohre 26 angeordnet, die mit dem Innenraum 35 des Fluidregisters 33 kommunizieren. Die Drainagerohre 26 weisen auf definierter Höhe die erwähnten Filter 27 auf, durch welche die Flüssigkeiten aus der Waschkolonne abgeführt werden.

Nach dem Anfahren der Waschkolonne 15 bildet sich ein kompaktes Kristallbett 16 aus. Das Kristallbett wird durch die aus dem hydraulischen Strömungsdruckverlust der Mutterlauge resultierende Kraft vorbei an den Filtern 27 in eine sogenannte Waschzone 36 unterhalb der Filter transportiert. Die Rückführung eines Teils der Mutterlauge zurück in die Kolonne mittels der Steuerpumpe 29 ermöglicht die Regelung dieser Transportkraft. Schwankungen des Kristallgehalts der zugeführten Suspension oder Änderungen der Kristallgrößenverteilung, die wesentlich den Strömungsdruckverlust beeinflussen, können dadurch kompensiert werden. Erkennbar sind solche Schwankungen durch Veränderungen der Lage der sogenannten Aufbau- bzw. Filtrationsfront, die in Figur 2 durch die strichpunktierte Linie 37 angedeutet ist. Die Filtrationsfront 37 zeichnet sich durch einen relativ abrupten Anstieg des Kristallgehalts aus.

Am unteren Ende der Waschkolonne werden die Kristalle mittels des Schabers 18 vom Kristallbett 16 abgetragen und in Reinproduktschmelze resuspendiert. Diese Suspension 38 wird in dem bereits im Zusammenhang mit Figur 1 beschriebenen Schmelzkreislauf 19 über den Wärmeübertrager 20 geführt, der die zum Schmelzen der Kristalle erforderliche Wärme in die Suspension einträgt. Der entsprechende Wärmeeintrag wird üblicherweise ebenfalls geregelt, wie in Figur 2 durch die Regeleinrichtung 39 schematisch angedeutet ist. Durch einen Temperaturregler der Regeleinrichtung 39 kann beispielsweise gewährleistet werden, daß der Wärmeübertrager 20 gerade soviel Energie in den Kreislauf einträgt, daß die Temperatur unmittelbar nach dem Wärmeübertrager 1 bis 5 K über dem Reinproduktschmelzpunkt liegt. Typischerweise werden 60 - 95 Gew.% der Schmelze als gereinigter Reinproduktstrom über das Produktventil 22 aus dem Schmelzkreislauf 19 abgeführt. Die restliche Produktschmelze durchströmt das Kristallbett in der durch den Pfeil 25 angedeuten Richtung,wodurch eine Gegenstromwäsche der Kristalle erfolgt und verläßt das Kristallbett durch den Filter 27.

Im stationären Betrieb stellt sich auf einer definierten Höhe der Waschzone 36 eine sogenannte Waschfront ein, die in Figur 2 durch die strichpunktierte Linie 40 angedeutet ist. Als Waschfront ist derjenige Ort in der Waschkolonne definiert, wo die höchsten Temperatur- und Konzentrationsgradienten auftreten. Auf Höhe der Waschfront findet in der das Kristallbett umgebenden Flüssigkeit ein Konzentrationsübergang von Mutterlaugenkonzentration (oberhalb der Waschfront) zu Reinschmelzekonzentration (unterhalb der Waschfront) statt. Die Temperatur des Festbetts oberhalb der Waschfront entspricht in etwa der Kristallisationstemperatur der Ausgangsflüssigkeit, während die Temperatur des Festbetts unterhalb der Waschfront der (höheren) Schmelztemperatur der Reinsubstanz entspricht. Im Bereich der Waschfront 40 kommt es daher zu einem Temperaturausgleich der kalten Kristalle mit der reinen Waschflüssigkeit, bei dem die Waschflüssigkeit teilweise oder vollständig rekristallisiert. Der nicht rekristallisierte Anteil der Waschflüssigkeit geht über die Filter 27 verloren. Die Waschfront 40 muß zur Erzielung einer adäquaten Reinigungswirkung in einer bestimmten Mindesthöhe oberhalb des Schabers 18 positioniert sein. Die Position der Waschfront stellt sich als dynamisches Gleichgewicht aus der mit dem Festbett 16 transportierten Mutterlauge und dem entgegenströmenden Waschflüssigkeitsstrom (Pfeil 25) ein.

Erfindungsgemäß sind zur Regelung der Lage der Aufbau- bzw. Filtrationsfornt 37 und der Lage der Waschfront 40 Zeilendetektoren vorgesehen. So ist bei der in Figur 2 dargestellten Ausführungs im Bereich der Aufbaufront 37 ein erstes lineares Array 41 von Remissionssonden 42 in der Seitenwand 43 der Waschkolonne 15 angeordnet. Jede der Remissionssonden 42 weist Sendefasern 44 auf, die mit einer Lichtquelle 45 verbunden sind, sowie Empfangsfasern 46, die mit einem Detektor 47 verbunden sind. Über die Sendefasern 44 wird Licht in das Kristallbett 16 eingestrahlt. Die Empfangsfasern 46 leiten das aus dem Kristallbett remitierte Licht zum Detektor 47, wo die Intensität des remittierten Lichtes gemessen und aufgezeichnet wird. Der Detektor 47 kann aber auch eine (nicht dargestellte) Einrichtung zur spektralen Analyse des remittierten Lichtes aufweisen. Eine Regeleinrichtung 48, die beispielsweise einen Computer umfaßt, wertet die vom Detektor 47 gelieferten Signale aus, bestimmt die Lage der Aufbaufront und steuert die Steuerstrompumpe 29 so, daß die Lage der Aufbaufront 37 auf einer bestimmten Sollposition gehalten wird. Stellt die Regeleinrichtung in extremen Betriebszuständen fest, daß eine Kompensation der Störungen über die Steuerpumpe nicht mehr möglich ist, so kann - je nach Regelungsstrategie - eine neue Sollposition der Aufbaufront 37 ermittelt werden, die unter den gegebenen Betriebsbedingungen eingeregelt werden kann. Dies ist möglich, weil durch die erfindungsgemäß vorgesehene Verwendung eines Zeilendetektors ein größerer Abschnitt der Kolonne lückenlos überwacht werden kann. Falls bei einer Betriebsstörung die Gefahr droht, dass die Aufbaufront diesen überwachten Abschnitt verlässt und eine Regelung nicht mehr möglich ist, kann auch ein Alarm ausgelöst oder die Anlage abgeschaltet werden.

Auf Höhe der Waschfront 40 ist ein entsprechendes zweites lineares Array 49 aus Remissionssonden 50 in der zylindrischen Kolonnenwand 43 angeordnet. Wieder wird über mit einer Lichtquelle 51 verbundenen Sendefasern 52 Licht in das Kristallbett 16 eingestrahlt und über Empfangsfasern 53 das aus dem Kristallbett remittierte Licht zu einem Detektor 54 geleitet. Aus den vom zweiten Remissionssondenarray 49 gelieferten Daten wird mittels einer Steuer- und Regeleinrichtung 55 die Lage der Waschfront 40 in der Kolonne 15 ermittelt und das Produktventil 22 so eingestellt, daß die Waschfront mit großer Genauigkeit auf einer bestimmten Sollposition gehalten wird. Stellt die Regeleinrichtung eine Abweichung der Waschfront 40 von der Sollhöhe fest, so wird über das Produktventil 22 der abgezogene Reinproduktstrom und damit auch die Waschflüssigkeitsmenge entsprechend geregelt (beispielsweise bei einem Absinken der Waschfront durch eine Erhöhung der Waschflüssigkeitsmenge).

Zur Einhaltung und Kontrolle der Produktreinheit kann man die Qualität des Reinprodukts aber auch kontinuierlich messen. Die Messung kann beispielsweise mittels eines optischen Extinktionssensors 56, der in einem geeigneten Spektralbereich arbeitet, direkt in der Produktleitung 23 oder einem Bypass erfolgen. Wenn die Qualitätsmessung (wie in Figur 2 dargestellt) in dem Schmelzkreislauf 19 erfolgt, kann sie auch für das Anfahren der Waschkolonne genutzt werden.

Zusätzlich zur Regelung der Lage der Waschfront 40 mittels des Zeilendetektors 49 kann eine an sich bekannte Regelung über Temperatursensoren 57 realisiert werden. In besonderen Fällen, in bei denen eine rein optische Bestimmung der Lage der Waschfront nicht zuverlässig möglich ist, kann dann auf die Daten der Temperatursensoren zurückgegriffen werden. Es ist natürlich auch möglich, nur die Lage der Aufbaufront über den optischen Zeilendetektor 42 zu bestimmen und die Lage der Aufbaufront konventionell ausschließlich über die Temperatursensoren 57 zu ermitteln.

In Fig. 3 ist eine zweite Ausführungsform der erfindungsgemäßen Vorrichtung dargestellt, bei der als optischer Zeilendetektor jeweils eine Zeilenkamera 58 zur Detektion der Filtrationsfront 37 und eine Zeilenkamera 59 zur Detektion der Waschfront 40 verwendet werden. Der grundsätzliche Aufbau der Waschkolonne der Figur 3 entspricht weitgehend dem der in Figur 2 dargestellten Waschkolonne. Die übereinstimmenden Bauteile sind mit denselben Bezugsziffern versehen und werden im folgenden nicht oder nur kurz erläutert. In der zylindrischen Seitenwand der Waschkolonne 15 sind auf Höhe der Waschfront bzw. der Filtrationsfront längliche Sichtfenster 60, 61 angeordnet, die von den Zeilenkameras 58, 59 abgebildet werden. Die Fenster 60, 61 können durch Beleuchtungseinrichtungen 62, 63 ausgeleuchtet werden. Auswerteeinrichtungen 64, 65 ermitteln aus den Daten der Zeilenkamera die Lage der Waschfront 40 und die Lage der Aufbaufront 37 und steuern die Steuerstrompumpe 29 bzw. das Produktventil 22 so, daß die Lage der Fronten auf den jeweiligen Sollpositionen gehalten wird.

Fig. 4 zeigt eine Variante der Ausführungsform der Fig. 3, bei der lediglich eine einzige CCD-Zeilenkamera 66 zur Detektion von Wasch- und Filtrationsfront verwendet wird. Dazu erstreckt das in der Wand 43 der Waschkolonne 15 vorgesehene Sichtfenster 67 über den größten Teil der Höhe der Waschkolonne. Wird mit einer solchen Vorrichtung beispielsweise ein axialer Abschnitt einer Länge von 2 m erfaßt und benutzt die CCD-Kamera einen Sensor mit 2048 linear angeordneten Pixeln, so ist noch eine Regelung der Lage der Waschfront bzw. der Lage der Aufbaufront mit einer Genauigkeit von ca. 1 bis 2 cm möglich. Über eine oder mehrere Lichtquellen 68, 69 wird das Fenster 67 ausgeleuchtet. Eine Steuerund Regelungseinrichtung 70 bestimmt aus den aufgezeichneten Daten die Lage der Waschfront bzw. der Aufbaufront und stellt die Steuerpumpe bzw. das Produktventil entsprechend ein.

Wenn in der vorliegenden Beschreibung von Zeilendetektoren die Rede ist, so hängt dies damit zusammen, dass für das erfindungsgemäße Regelungsverfahren lediglich eine quasi eindimensionale Messung entlang der Achse der Waschkolonne erforderlich ist. Sebstverständlich können ohne Nachteile auch Flächendetektoren, beispielsweise herkömmliche Digitalkameras mit zweidimensionalen CCD-Chips zur Realisierung des erfindungsgemäßen Verfahrens herangezogen werden. Die Ermittlung des zu analysierenden Bildbereichs, also beispielsweise der Bereich des Sichtfensters der Kolonne 15, kann durch kommerzielle Bildverarbeitungssoftware erfolgen.

## Patentansprüche

1. Verwendung eines optischen Zeilendetektors zur Regelung einer Waschkolonne in einem Schmelzkristallisationsprozeß

2. Verfahren zur Regelung einer Waschkolonne in einem Schmelzkristallisationsprozeß, bei dem man
eine Suspension, die in einer Mutterlauge suspendierte Kristalle einer zu reinigenden Substanz enthält, kontinuierlich in eine Waschkolonne leitet,
in der Waschkolonne ein Kristallbett der zu reinigenden Substanz ausbildet, wobei das Kristallbett eine Aufbaufront aufweist, an der sich kontinuierlich Kristalle der eingeleiteten Suspension anlagern,
an dem der Aufbaufront gegenüberliegenden Ende des Kristallbetts kontinuierlich Kristalle abträgt, die abgetragenen Kristalle aufschmilzt, einen Teil der Schmelze als Reinproduktstrom abführt und einen anderen Teil der Schmelze als Waschflüssigkeitsstrom gegen die Transportrichtung der Kristalle durch das Kristallbett leitet, wobei sich im Kristallbett eine Waschfront ausbildet,
**dadurch gekennzeichnet, daß**
man die Lage der Aufbaufront und/oder der Waschfront in der Waschkolonne mittels wenigstens eines optischen Zeilendetektors regelt.

3. Verfahren gemäß Anspruch 2, **dadurch gekennzeichnet, daß** man die Lage der Waschfront mittels eines optischen Zeilendetektors misst und durch Steuerung des abgezogenen Reinproduktstroms auf eine Sollposition regelt.

4. Verfahren gemäß einem der Ansprüche 2 oder 3, wobei man eine hydraulische Waschkolonne verwendet, **dadurch gekennzeichnet, daß** man die Lage der Aufbaufront mittels eines optischen Zeilendetektors misst und durch Steuerung des hydrodynamischen Drucks der weder Mess-noch Regelgröße ist, bzw. Steuerung der oberhalb der Aufbaufront zugeführten Flüssigkeistmenge in der Waschkolonne auf eine Sollposition regelt.

5. Verfahren gemäß einem der Ansprüche 2 bis 4, **dadurch gekennzeichnet, daß** man als Zeilendetektor wenigstens ein lineares Array von faseroptischen Remissionssonden verwendet.

6. Verfahren gemäß einem der Ansprüche 2 bis 4, **dadurch gekennzeichnet, daß** man als Zeilendetektor wenigstens eine Zeilenkamera, insbesondere eine CCD-Zeilenkamera, verwendet.

7. Vorrichtung zur Trennung von Kristallen und Mutterlauge in einem Suspensionskristallisationsprozeß, insbesondere zur Durchführung des Verfahrens nach einem der Ansprüche 1 bis 6, mit
einer Waschkolonne (15), die wenigstens eine Zuleitung (13) für eine Kristallsuspension, Filtrationsmittel (26,27) zum Abführen von Mutterlauge und Mittel (18) zum Abtragen von Kristallen aus einem Kristallbett aufweist,
Mitteln (20) zum Aufschmelzen der abgetragenen Kristalle,
einer Abzugseinrichtung (22,23) zum Abführen eines Teils der Schmelze als Reinproduktstrom,
wenigstens einer Meßeinrichtung zur Bestimmung der Lage einer Aufbaufront(37) und/oder einer Waschfront (40) des Kristallbetts in der Waschkolonne,
Mitteln zur Regelung der Lage der Aufbaufront und/oder der Lage der Waschfront,
**dadurch gekennzeichnet, daß**
die Meßeinrichtung wenigstens einen optischen zeilendetektor umfaßt.

8. Vorrichtung gemäß Anspruch 7, **dadurch gekennzeichnet, daß** der optische Zeilendetektor ein lineares Array (41,49) von faseroptischen Remissionssonden (42,50) umfaßt, die in einem Mantel (43) der Waschkolonne (15) angeordnet sind.

9. Vorrichtung gemäß Anspruch 7, **dadurch gekennzeichnet, daß** in einem Mantel (43) der Waschkolonne (15) wenigstens ein transparentes Fenster (60,61,67) angeordent ist, und daß der optische Zeilendetektor wenigstens eine Zeilenkamera (58,59,66), insbesondere eine CCD-Zeilenkamera, umfaßt.

10. Vorrichtung gemäß Anspruch 9, **dadurch gekennzeichnet, daß** der optische Zeilendetektor eine Farb-CCD-Zeilenkamera umfaßt.

## Claims

1. The use of an optical line detector for regulating a wash column in a melt crystallization process.

2. A method for regulating a wash column in a melt crystallization process in which
a suspension, which contains, suspended in a mother liquor crystals of a substance to be purified, is passed continuously into a wash column,
a crystal bed of crystals of the substance to be purified forms in the wash column, the crystal bed having a build-up front at which crystals of the suspension passed in continuously accumulate,
crystals are continuously removed at that end of the crystal bed which is opposite to the build-up front, the crystals removed are melted, a part of the melt is removed as pure product stream and another part of the melt is passed as a wash liquid stream through the crystal bed in the direction opposite to the transport direction of the crystals, a wash front forming in the crystal bed, wherein the position of the build-up front and/or of the wash front in the wash column is regulated by means of at least one optical line detector.

3. A method as claimed in claim 2, wherein the position of the wash front is measured by means of an optical line detector and is regulated to a setpoint position by controlling the pure product stream taken off.

4. A method as claimed in either of claims 2 and 3, a hydraulic wash column being used, wherein the position of the build-up front is measured by means of an optical line detector and is regulated to a setpoint position in the wash column by controlling the hydrodynamic pressure, which is neither a measured variable nor a control variable, or controlling the amount of liquid fed in above the build-up front.

5. A method as claimed in any of claims 2 to 4, wherein the line detector used comprises at least one linear array of fiber-optic reflection probes.

6. A method as claimed in any of claims 2 to 4, wherein the line detector used comprises at least one line camera, in particular a CCD line camera.

7. An apparatus for separating crystals and mother liquor in a suspension crystallization process, in particular for carrying out the method as claimed in any of claims 1 to 6, comprising
a wash column (15) which has at least one feed line (13) for a crystal suspension, filtration means (26, 27) for removing mother liquor and means (18) for removing crystals from a crystal bed,
means (20) for melting the crystals removed,
a take-off means (22, 23) for removing a part of the melt as pure product stream,
at least one measuring means for determining the position of a build-up front (37) and/or of a wash front (40) of the crystal bed in the wash column,
means for regulating the position of the build-up front and/or the position of the wash front,
**characterized in that**
the measuring means comprises at least one optical line detector.

8. An apparatus as claimed in claim 7, wherein the optical line detector comprises a linear array (41, 49) of fiber-optic reflection probes (42, 50) which are arranged in a wall (43) of the wash column (15).

9. An apparatus as claimed in claim 7, wherein at least one transparent window (60, 61, 67) is arranged in a wall (43) of the wash column (15), and wherein the optical line detector comprises at least one line camera (58, 59, 66), in particular a CCD line camera.

10. An apparatus as claimed in claim 9, wherein the optical line detector comprises a color CCD line camera.

## Revendications

1. Utilisation d'un détecteur optique multi-rangée pour la régulation d'une colonne de lavage dans un procédé de cristallisation en fusion.

2. Procédé de régulation d'une colonne de lavage dans un procédé de cristallisation en fusion, dans lequel
on fait passer, en continu dans une colonne de lavage, une suspension qui contient des cristaux, en suspension dans une liqueur mère, d'une substance à purifier,
on forme, dans la colonne de lavage, un lit de cristaux de la substance à purifier, le lit de cristaux présentant un front d'accumulation, où les cristaux de la suspension introduite se déposent en continu,
on retire en continu les cristaux à l'extrémité opposée du lit de cristaux, on fait fondre les cristaux retirés, on soutire une partie de la masse fondue en tant que courant de produits purs et on conduit une autre partie de la masse fondue en tant que courant de liquide de lavage à contre-courant de la direction de transport des cristaux à travers le lit de cristaux, ce qui engendre la formation d'un front de lavage dans le lit de cristaux, **caractérisé en ce que**
l'on règle la position du front d'accumulation et/ou du front de lavage dans la colonne de lavage au moyen d'au moins un détecteur optique multi-rangée.

3. Procédé selon la revendication 2, **caractérisé en ce que** l'on mesure la position du front de lavage au moyen d'un détecteur optique multi-rangée et **en ce qu'**on la règle à une position de consigne en régulant le courant de produits purs soutiré.

4. Procédé selon l'une quelconque des revendications 2 ou 3, dans lequel on utilise une colonne de lavage hydraulique, **caractérisé en ce que** l'on mesure la position du front d'accumulation au moyen d'un détecteur optique multi-rangée et **en ce qu'**on la règle à une position de consigne en régulant la pression hydrodynamique, qui n'est ni une variable mesurée, ni une variable réglée, ou en régulant la quantité de liquide introduite au-dessus du front d'accumulation dans la' colonne de lavage.

5. Procédé selon l'une quelconque des revendications 2 à 4, **caractérisé en ce que** l'on utilise, en tant que détecteur multi-rangée, au moins une matrice linéaire de capteurs de réflexion à fibres optiques.

6. Procédé selon l'une quelconque des revendications 2 à 4, **caractérisé en ce que** l'on utilise, en tant que détecteur multi-rangée, au moins une caméra linéaire, en particulier une caméra CCD linéaire.

7. Dispositif pour la séparation de cristaux et de la liqueur mère dans un procédé de cristallisation en suspension, en particulier pour la réalisation du procédé selon l'une quelconque des revendications 1 à 6, comprenant
une colonne de lavage (15) qui présente au moins une alimentation (13) pour une suspension de cristaux, des moyens de filtration (26, 27) pour évacuer la liqueur mère et des moyens (18) pour retirer les cristaux d'un lit de cristaux,
des moyens (20) pour faire fondre les cristaux retirés,
un dispositif de soutirage (22, 23) pour'évacuer une partie de la masse fondue en tant que courant de produits purs,
au moins un dispositif de mesure pour déterminer la position d'un front d'accumulation (37) et/ou d'un front de lavage (40) du lit de cristaux dans la colonne de lavage,
des moyens pour régler la position du front d'accumulation et/ou du front de lavage,
**caractérisé en ce que**
le dispositif de mesure comprend au moins un détecteur optique multi-rangée.

8. Dispositif selon la revendication 7, **caractérisé en ce que** le détecteur optique, multi-rangée comprend une matrice linéaire (41, 49) de capteurs de réflexion à fibres optiques (42, 50) qui sont disposés dans une enveloppe (43) de la colonne de lavage (15).

9. Dispositif selon la revendication 7, **caractérisé en ce qu'**au moins une fenêtre transparente (60, 61, 67) est disposée dans une 'enveloppe (43) de la colonne de lavage (15) et que le détecteur optique multi-rangée comprend au moins une caméra linéaire (58, 59, 66), en particulier une caméra CCD linéaire.

10. Dispositif selon la revendication 9, **caractérisé en ce que** le détecteur optique multi-rangée comprend une caméra couleur CCD linéaire.
